# EUROPEAN PATENT APPLICATION

(11) **EP 4 005 421 A1**
(43) Date of publication of application: **01.06.2022**
(21) Application number: 20852990.9
(22) Date of filing: 30.04.2020
(51) Int. Cl.: A24F 47/00

(54) **NEW TYPE OF VAPORIZATION CORE**

(30) Priority: 13.08.2019 CN 201910742101
(71) Applicant: Shanghai QV Technologies Co., Ltd., Shanghai 201204 (CN)
(72) Inventor: PENG, Xiaofeng, Shanghai 200050 (CN); PENG, Qiwen, Shanghai 200050 (CN)
(74) Representative: Nederlandsch Octrooibureau
(86) International application number: PCT/CN2020/088397
(87) International publication number: WO 2021/027338

(57) **Abstract**

The present invention relates to the field of atomization applications. More specifically, the invention discloses a novel atomization core, comprising a core substrate and a heating body on the core substrate, wherein the core substrate is made of a dense material, with e-liquid transferring perforations distributed in the substrate; the diameter of the e-liquid transferring perforations is 1-250 µm; the wall spacing between two adjacent e-liquid transferring perforations is less than 500 µm; and the porosity of the dense ceramics is less than 30%. The disclosed novel atomization core can perform not only similar in-situ atomizing by controllable fluidic channels formed in the dense substrate but also quantified or dose controlled vaping by quantified flow control, further uniform vaping by controlled atomizing nucleation process and particle growing process. At the same time, ceramic powder and relatively toxic and harmful substances caused by the porous ceramic itself are avoided from entering the atomized aerosol, thereby a safer quantitative atomization is achieved.

## Description

### Technical Field

The invention relates to atomization applications. More specifically, the invention relates to a novel atomization core.

### Background Art

At present, electrical resistance heating is normally employed in e-cigarettes and some medical atomizers to heat liquids to generate aerosol. There are following four types in general:
First, glass fiber rope plus heating wire: the most common e-cigarette atomizer generally winds the resistance heating wire on the fiber rope used for transferring the liquid. The glass fiber rope is used as the main transferring material because of its firm selvage, high temperature resistance, strong liquid absorption, and fast transfer speed. However, the biggest disadvantage of glass fiber rope is that it is easy to fall off and produce floes. Furthermore, the position of the heating wire is varied when the heating wire is wound around the fiber rope, the surface of the heating wire is differently exposed to the outside of the fiber rope, which results in low consistency of the atomizing device, low atomizing efficiency, and dry burning.

Second, cotton plus heating wire: around 2013, cotton began to replace glass fiber rope as the main transferring material. Compared with glass fiber rope, it is safer and delivers a flavor more authentical to that of tobacco through the e-liquid. Its development has gone from absorbent cotton and organic cotton to professional e-cigarette cotton such as highest grade long-staple cotton. At present, cotton plus heating wire is still the mainstream in the market, but sugar in the e-liquid will be adsorbed on the surface of heating wire to form what we usually call carbon deposit, which leads to the darkening of cotton and is also easy to generate harmful and potential harmful constituents (HPHC) in the aerosol.

Third, ceramic atomization core: the development of e-cigarette has led to the emergence of various transferring materials. Porous ceramic transferring materials have become popular for e-cigarettes. There are mainly two kinds of ceramic atomization cores on the market: one is to embed heating wires in a porous ceramic body, eg. CCell; the other is to screen print a layer of conductive heating coating on the porous ceramic, e.g. Feelm and Silmo. The pores of the porous ceramic are dispersed in various sizes, resulting in easily coking or dry burning of some liquid components during vaping, or leakage of liquid due to large perforations. CN20188001973.3 has disclosed that a 0.5-5 µm thick titanium-zirconium alloy film and a 0.1-1 µm thick Au-Ag alloy protective film are sputtered and deposited on the porous ceramic. At this thickness, the film quality is inevitably affected by the surface roughness of the porous ceramic.

Fourth, other atomization cores: for example, CN201620757596.4, CN201810009220.9 and CN201910229470.8 have disclosed monocrystalline silicon-based MEMS atomization cores, which are expected to solve the problems of inconsistent atomizing temperature and flavor change caused by direct contact between the heating surface and the e-liquid. A micro-perforation plate with micro-perforation array is used to control the liquid flow. The diameter of the micro fluidic channels is 10 to 500 µm, and those of the micro-perforation channels are 500 to 1000 µm. The metal films are one or more of Ti/Pt/Au, TiW/Au, Al, Cr or Pt/Au with a thickness of 200 to 500nm. However, the system reliability of such devices is still at stake. Another example is CN201821218626.X and CN201810855337.9, which describe an atomizer of capillary array using stainless-steel medical tubes and glass tubes with inner diameters of 0.01-0.1mm as capillaries array. The external stainless-steel sheet is directly heated, thus similarly avoiding the contact between the heating body and e-liquid. The effective atomization area where the fluid passes through reaches up to 50%. These patents claim to have overcome the shortcomings of ceramic heating bodies, thus achieving atomized e-cigarette more authentical to traditional cigarettes. However, the processing and assembly of micro-tubes pose certain safety risks for powder and other particles to enter the aerosol.

In terms of safety, although the above four atomization methods have greatly reduced the harmful ingredients comparing to traditional cigarettes, there are still certain traces of toxic and harmful substances. In particular, due to the manufacturing process and structural features of the porous ceramic, some adhered buried powder particles or ceramic particles themselves will inevitably be introduced into the aerosol. In addition, due to crystal phase segregation, heavy metal impurities are concentrated on the surface of crystal grains, which are easily leached into the atomized liquid. At present, experiments have proved that trace amounts of heavy metals can be detected in the aerosols of some e-cigarettes with ceramic atomization cores.

### Summary of the Invention

The present invention aims to overcome the weaknesses in the above-mentioned prior arts and provides a novel atomization core, which not only realizes safer atomization, but also allows, with precise design, dose-control atomization and uniform atomization without coking or particle emission.

For the above purposes, the novel atomization core disclosed by the present invention comprises a core substrate and a heating body on the core substrate, wherein the core substrate is made of a dense material, with e-liquid transferring perforations distributed in the core substrate, a diameter of the e-liquid transferring perforations is 1-250 µm, and a wall spacing between two adjacent e-liquid transferring perforations is less than 500 µm.

The dense material may be one of the following materials: monocrystalline or polycrystalline materials, high temperature resistant and thermal shock resistant glasses, or dense ceramics. Preferably, the monocrystalline materials may be monocrystalline alumina and monocrystalline silicon; and polycrystalline silicon materials and the like; the high temperature resistant and thermal shock resistant glasses may be quartz glass, borosilicate glass or aluminosilicate glass; and the dense ceramic may be silica, alumina, zirconia, zinc oxide, silicon carbide, diatomite, mullite, zirconite or apatite with a relative density exceeding 70%.

Preferably, the porosity of the dense ceramic is less than 30%; more preferably, the porosity of the dense ceramic is less than 10%.

Preferably, the heating body is a thin film/coating or a metal heating body.

Preferably, the heating body is coated or screen printed, vapor deposited, liquid deposited or directly bonded to the substrate of the atomization core.

Preferably, the thickness of the heating body is, less than 100 µm if coated or screen-printed, 5 µm or less if deposited, or less than 50 µm if bonded.

Preferably, the heating body is selected from biocompatible films such as titanium, tantalum and alloy thereof, or titanium/tantalum oxide films, or metal foils bonded with the substrate of the atomization core. Optionally, a protective passive film is further provided on the heating body as needed.

The diameter of the e-liquid transferring perforations is 150 µm or less, preferably between 25 µm and 120 µm, and more preferably 80 µm or less.

Preferably, the wall spacing between two adjacent e-liquid transferring perforations is below 250 µm, preferably below 150 µm, and more preferably below 100 µm.

Preferably, the e-liquid transferring perforations are made by extrusion molding, injection molding, compression molding, 3D printing, laser processing or mechanical drilling.

By forming controllable fluidic channels in the substrate, the disclosed novel atomization core can perform not only approximately in-situ atomizing, but also accurately dose-control vaping by quantifying the fluidic channels, and also uniform atomizing by maximally controlling the nucleation and growth processes of atomized particles. More importantly, the substrate is no longer porous ceramic, and the vaping interface of the whole atomization process is very stable and safe, ceramic particle emission and relatively toxic and harmful substances in the porous ceramic based atomizer are fully avoided to atomized aerosol, thereby a safer, more uniform and quantitative vaping is achieved.

### Brief Description of the Drawings

Figure 1 is a schematic diagram of the structure of a substrate of the atomization core according to Embodiment 1 of the present invention;
Figure 2 is a schematic diagram of the structure of a substrate of the atomization core according to Embodiment 2 of the present invention;

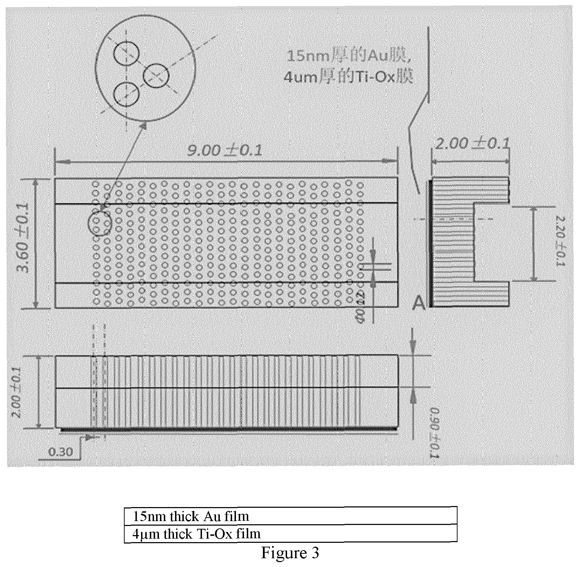

Figure 3 is a schematic diagram of the structure of a substrate of the atomization core according to Embodiment 4 of the present invention.

### Detailed Description of the Embodiments

In order to make the objective, technical solutions and advantages of the present invention clearer, detailed description of the present invention is further given below, with reference to the accompanying drawings and embodiments. Additional features and advantages of the present invention will be provided in part in the following description, and will be clearer in part from the following description, or may be experienced by practice of the present invention. It is to be understood that the following description is exemplary only and is not restrictive of the present invention.

The novel atomization core disclosed by the present invention comprises a core substrate and a heating body on the core substrate, wherein the core substrate is made of a dense material, with e-liquid transferring perforations distributed in the substrate, a diameter of the e-liquid transferring perforations is 1-250 µm, and a wall spacing between two adjacent e-liquid transferring perforations is below 500 µm. The dense material may be one of the following materials: monocrystalline alumina or other monocrystalline or polycrystalline materials, high-temperature resistant and thermal shock resistant glasses, and dense ceramics. Preferably, the high temperature resistant and thermal shock resistant glasses may be quartz glass, borosilicate glass or aluminosilicate glass, and the dense ceramics may be silica, alumina, zirconia, zinc oxide, silicon carbide, diatomite, mullite, zirconite or apatite with a relative density exceeding 70%. The porosity of the dense ceramic is less than 30%. The heating body is a thin film/coating or metal heating body, which is coated or screen printed, vapor deposited, liquid deposited or directly bonded to the core substrate. The e-liquid transferring perforations are made by extrusion molding, injection molding, compression molding, 3D printing, laser processing or mechanical drilling.

In the present invention, the dense material and its thickness, the sizes and positions of the e-liquid transferring perforations are critical to control the atomized aerosol, as the performance of the aerosol depends on precise control and uniformity of the same. The sizes of the e-liquid transferring perforations are critical to aerosol chemical compositions and particle size control, and they are also critical to prevent coking at low temperature. According to the invention, the common use of porous ceramic is avoided, and the overall strength of the atomization core material is greatly improved, thereby ceramic particles emissions into the aerosol and further damaging the lungs of the vapors are fully avoided.

The atomization core of the present invention overcomes the disadvantages of currently used porous ceramics, including uncontrollable porosity, uneven pore sizes and distribution, rough surface, inconsistent atomizing interface caused by grain boundary segregation in the preparation of porous ceramics. With the understanding of the atomizing mechanism and the atomizing interface, the mechanism of uniform and quantitative atomization is established, and thereby both the particles size distributions and chemical components of the atomized aerosol are improved. Consequently, the taste/flavor authentic, taste consistency from first puff to last puff, and the taste satisfactions are greatly improved. In addition, the size and the number of the e-liquid transferring perforations of the present invention may be tailored according to the characteristics of the e-liquid. Thus some disadvantages in the conventional porous ceramic based coils are fully avoided such as low temperature coking of some ingredients in the e-liquid because of the unmatching between pore size and some certain chemical ingredients. Thanks to the control of vaping interface and mechanism, some chemical reaction and thermal decomposition in the atomization process are greatly inhibited. Thus the HPHC (harmful and potential harmful constituents) and heavy metals in the aerosol are greatly reduced. Furthermore, the ceramic particle emission is also fully eliminated.

Preferably, the porosity of the dense material of the present invention is less than 10%; the thickness of the heating body is less than 100 µm if coated or screen-printed, 5 µm or less if vapor deposited, or less than 50 µm if bonded.

Preferably, the heating body of the present invention is selected from biocompatible materials such as titanium, tantalum et al, or alloys thereof, or titanium/tantalum oxide films, or metal foils bonded with the core substrate. The heating body may also be other heat-resistant conductive compounds or mixture films. A protective passive film may be further provided on the heating body as needed.

The diameters of the e-liquid transferring perforations are 150 µm or less, preferably between 25 µm and 120 µm, and more preferably 80 µm or less

Preferably, the wall spacing between two adjacent e-liquid transferring perforations is below 250 µm, preferably below 150 µm, and more preferably below 100 µm.

In the present invention, thanks to the control of the fluidic channels and in-situ heating, the atomization nucleation and the dynamic growth after nucleation are more accurately controlled, so that the particle size and composition, quantity/volume and temperature of the atomized aerosol may be controlled or tailored according to specific atomization requirements, and the nicotine transmission efficiency can be improved to a certain extent. The outlet of each e-liquid transferring perforation is critical to the origination of atomization nucleation. Of course, e-liquid will also be spread onto the heating surface. The wall spacing between the two adjacent perforations needs to be controlled below 250 µm, which will greatly mitigate the risks that the e-liquid fails to completely cover the heating surface, or will not prevent the e-liquid from covering the heating surface in the atomization process, thus either dry burning or local over-high temperature is fully avoided. Hence in-situ atomization or in-situ vaping can be defined in the present invention. At present, the vast majority of atomizing devices employs ex-situ heating through heat conduction, resulting in uneven temperatures, which is also the main reason why HPHC cannot be completely eliminated.

Technical features involved in various embodiments of the present invention can be combined with each other as long as they do not conflict with each other.

### Embodiment 1

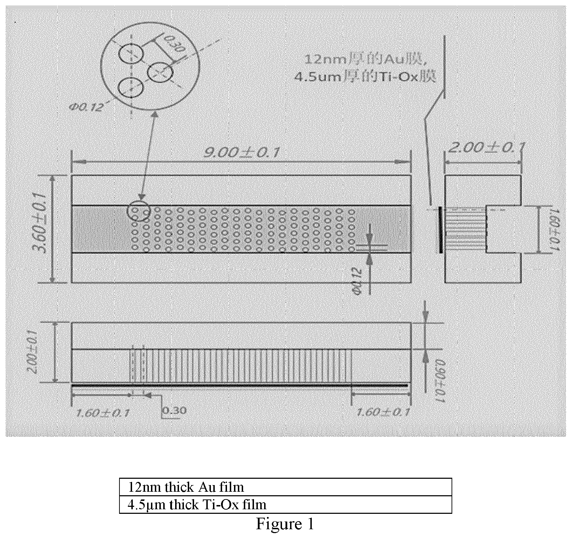

The substrate is made of monocrystalline alumina. After processing by CNC machining into its shape and dimensions, zoom laser is employed to form an array of perforations in it, with perforation diameters of 120 µm, 100 µm, 80 µm or 60 µm, and wall spacing between two adjacent perforations of 250 µm, 200 µm, 150 µm or 100 µm, respectively. The array of perforations can be a close-packed triangular or rectangular shape or other shapes. After that, a titanium or tantalum oxide film (4.5 µm titanium oxide film in Figure 1) with a thickness ranging from 0.35 µm to 5 µm is deposited by sputtering or electron beam evaporation. And the thickness is directly related to the oxygen content in the thin film. As to different oxygen content in the thin films or thin films with low oxygen content, a passive film is further deposited thereon, such as an Au film with about 12 nm thickness (as shown in Figure 1). Then electrodes are formed with safe conductive paste on both ends of the substrate and connected to the battery. The thickness of each film depends on the design of the resistance and atomization power. The film deposited between the perforation walls provides a uniform temperature field and a uniform nucleation center, and forms controllable liquid fluidic and air fluidic channels during atomization. Consequently the volume and the properties of the atomized aerosol are well controlled to achieve better nicotine delivery efficiency and various aerosol satisfactions. The uniform temperature field is resulted from the design of heating element, i.e. the design of screen-printed coating or deposited film or metal foil, which is directly controlled by the uniformity of wall spacing. The non-porous areas are the heating surface, and the controllable liquid and air flow refer to the control of the fluidic channels and the interface of atomization nucleation. For different kinds of e-liquids and other liquids, uniform atomization is achieved without coking or ceramic particle emission. Figure 1 shows one example.

### Embodiment 2

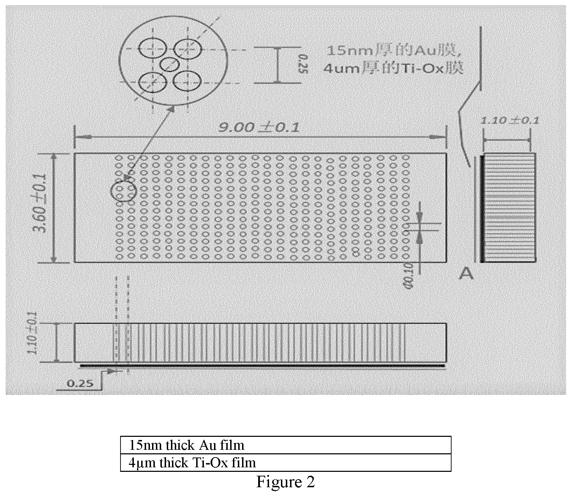

The substrate is made of monocrystalline alumina. After processing by CNC machining into its shape and dimensions, zoom laser is employed to form an array of perforations in it, with perforation diameter of 100 µm and wall spacing between two adjacent perforations of 200 µm. The array of perforations can be arranged in a close-packed triangular or rectangular shape or other shapes. After that, a titanium or tantalum oxide film (4 µm titanium oxide film in Figure 2) with a thickness ranging from 0.35 µm to 5 µm is deposited by sputtering or electron beam evaporation. As the thickness is directly related to the oxygen content in the thin film. As to different oxygen content in the thin films or thin films with low oxygen content, a passive film is further deposited thereon, such as an Au film with about 15 nm (as shown in Figure 2). Then electrodes are formed with safe conductive paste on both ends of the substrate and connected to the battery. The thickness of each film depends on the resistance and atomization power required. The film deposited between the perforation walls forms a uniform temperature field and a uniform nucleation center, and forms controllable fluid fluidic and air fluidic channels during atomization. Consequently the volume and the properties of the atomized aerosol are well controlled to achieve better nicotine delivery efficiency and various aerosol satisfactions. The uniform temperature field is resulted from the design of heating element, i.e. the design of screen-printed coating or deposited film or metal foil, which is directly controlled by the uniformity of wall spacing. The non-porous areas are the heating surface, and the controllable liquid and air flow refer to the control of the fluidic channels and the interface of atomization nucleation. For different kinds of e-liquids and other liquids, uniform atomization is achieved without coking, ceramic particle emission or any heavy metals.

### Embodiment 3

The substrate is made of transparent quartz glass. After processing by CNC machining into its shape and dimensions, zoom laser is employed to form an array of perforations in it, with perforation diameters of 120 µm and 80 µm, and wall spacing controlled at 200 µm and 150 µm, respectively. The array of perforations is arranged in a close-packed triangular shape, or can be arranged in a close-packed rectangular shape or other shapes. After that, a titanium or tantalum oxide film with a thickness ranging from 0.35 µm to 5 µm is formed by sputtering or electron beam evaporation. The thickness is directly related to the oxygen content in the thin film. As to different oxygen content in the thin films or thin films with low oxygen content, a passive film is further deposited thereon, such as an Au film with about 15 nm. Then electrodes are formed with safe conductive paste on both ends of the substrate and connected to the battery. The thickness of each film depends on the resistance and atomization power required. The film deposited between the perforation walls forms a uniform temperature field and a uniform nucleation center, and forms controllable liquid fluidic and air fluidic channels during atomization. Consequently the volume and the properties of the atomized aerosol are well controlled to achieve better nicotine delivery efficiency and various aerosol satisfactions. The uniform temperature field is resulted from the design of heating element, i.e. the design of deposited film or metal foil, which is directly controlled by the uniformity of wall spacing. The non-porous areas are the heating surface, and the controllable liquid and air flow refer to the control of the fluidic channels and the interface of atomization nucleation. For different kinds of e-liquids and other liquids, uniform atomization is achieved without coking or ceramic particle emission.

### Embodiment 4

The substrate is dense zirconia ceramic, prepared by 3D printing. The array of perforations is also formed in 3D printing process. The perforation diameters are 120 µm and 100 µm respectively, and the wall spacing between the two adjacent perforations is controlled at 180 µm. The array of perforation is arranged in a close-packed triangular shape. After that, a titanium or tantalum oxide film with a thickness ranging from 0.35 µm to 5 µm is deposited by sputtering or electron beam evaporation. The thickness is directly related to the oxygen content in the film. As to different oxygen content in the thin films or thin films with low oxygen content, a passive film is further deposited thereon, such as an Au film with about 15 nm. Then electrodes are formed with safe conductive paste on both ends of the substrate and connected to the battery. The thickness of each film depends on the resistance and atomization power required. The film deposited between the perforation walls forms a uniform temperature field and a uniform nucleation center, and forms controllable liquid fluidic and air fluidic channels during atomization. Consequently the volume and the properties of the atomized aerosol are well controlled to achieve better nicotine delivery efficiency and various aerosol satisfactions. The uniform temperature field is resulted from the design of heating element, i.e. the design of deposited film or metal foil, which is directly controlled by the uniformity of wall spacing. The non-porous areas are the heating surface, and the controllable liquid and air flow refer to the control of the fluidic channels and the interface of atomization nucleation. For different kinds of e-liquids and other liquids, uniform atomization is achieved without coking or ceramic particle emission.

The novel atomization core disclosed by the present invention can be used not only for e-cigarettes, but also for medical atomization (e.g. atomizer/nebulizer for pain relief and asthma relief) and entertainment atomization.

A person skilled in the art can easily understand that the foregoing descriptions are merely preferred embodiments of the present invention, but are not intended to limit the present invention. Any modifications, equivalent replacements, or improvements made within the spirit and principle of the present invention shall fall within the protection scope of the present invention.

## Claims

1. A novel atomization core, comprising a core substrate and a heating body on the core substrate, **characterized in that**, the core substrate is made of a dense material, with e-liquid transferring perforations distributed in the core substrate, a diameter of the e-liquid transferring perforations is 1-250 µm, and a wall spacing between two adjacent e-liquid transferring perforations is less than 500 µm.

2. The novel atomization core according to Claim 1, **characterized in that**, the dense material comprises a monocrystalline or polycrystalline material, a high temperature resistant and thermal shock resistant glass, and a dense ceramic, wherein, the monocrystalline material includes monocrystalline alumina and monocrystalline silicon, polycrystalline silicon materials and the like; the high temperature resistant and thermal shock resistant glass includes quartz glass, borosilicate glass or aluminosilicate glass; and the dense ceramic includes silica, alumina, zirconia, zinc oxide, silicon carbide, diatomite, mullite, zirconite and apatite with a relative density exceeding 70%.

3. The novel atomization core according to Claim 1 or Claim 2, **characterized in that**, a porosity of the dense ceramic is less than 30%.

4. The novel atomization core according to Claim 1, **characterized in that**, the heating body is a thin film/coating or a metal heating body.

5. The novel atomization core according to Claim 1 or Claim 4, **characterized in that**, the heating body is coated or screen printed, vapor deposited, liquid deposited or directly bonded to the core substrate.

6. The novel atomization core according to Claim 1 or Claim 4, **characterized in that**, a thickness of a material of the heating body is, less than 100 µm if coated or screen-printed, 5 µm or less if deposited, or less than 50 µm if bonded.

7. The novel atomization core according to Claim 1 or Claim 4, **characterized in that**, the heating body is selected from biocompatible films of titanium, tantalum and alloys thereof, or titanium/tantalum oxide films containing oxygen atoms, or metal foils bonded with the core substrate.

8. The novel atomization core according to Claim 1, **characterized in that**, the diameter of the e-liquid transferring perforations is 150 µm or less, preferably between 25 µm and 120 µm, and more preferably 80 µm or less.

9. The novel atomization core according to Claim 1 or Claim 8, **characterized in that**, the wall spacing between two adjacent e-liquid transferring perforations is below 250 µm, preferably below 150 µm, and more preferably below 100 µm.

10. The novel atomization core according to Claim 1, **characterized in that**, the e-liquid transferring perforations are made by extrusion molding, injection molding, compression molding, 3D printing, laser processing or mechanical drilling.
